# EUROPEAN PATENT SPECIFICATION

(11) **EP 3 337 434 B1**
(45) Date of publication and mention of the grant of the patent: **18.10.2023**
(21) Application number: 16837928.7
(22) Date of filing: 19.08.2016
(51) Int. Cl.: A61F 6/08, A61F 6/12, A61F 2/00

(54) **FLEXIBLE CONE-SHAPED INTRA-VAGINAL SUPPORT DEVICE**
FLEXIBLE KEGELFÖRMIGE INTRAVAGINALE STÜTZVORRICHTUNG
DISPOSITIF DE SUPPORT INTRA-VAGINAL SOUPLE ET EN FORME DE CÔNE

(30) Priority: 20.08.2015 US 201562283092 P
(43) Date of publication of application: 27.06.2018
(73) Proprietor: Watkins-Conti Products, Inc., Edmond, Oklahoma 73034 (US)
(72) Inventor: CONTI, Allison, Edmond, Oklahoma 73034 (US)
(74) Representative: Crease, Devanand John
(86) International application number: PCT/US2016/047859
(87) International publication number: WO 2017/031456

(56) References cited:
- WO-A1-2007/082341
- WO-A1-2015/041353
- WO-A1-2016/149317
- DE-U1-202009 008 893
- US-A- 1 996 242
- US-A- 2 534 900
- US-A- 3 845 766
- US-A- 3 845 766
- US-A- 5 827 248
- US-A1- 2003 149 334
- US-A1- 2008 077 097
- US-A1- 2008 149 109
- US-A1- 2009 283 099

## Description

### CROSS-REFERENCE TO RELATED APPLICATIONS

### TECHNICAL FIELD

The present disclosure relates in general to a flexible and non-absorbent vaginal insert device for use in improving symptoms associated with pelvic organ prolapse and urinary incontinence when the device is inserted, and more particularly to a vaginal insert device that is easier to insert and remove.

### BACKGROUND

Stress Urinary Incontinence (SUI) and Pelvic Organ Prolapse (POP) are growing problems globally that not only cost health care systems large amounts of money, but severely degrade the quality of life of tens of millions of women in the United States alone. Although surgical solutions can succeed in ameliorating symptoms associated with SUI and POP, surgery is not without risks and complications and may even leave the patient in a worse situation than before treatment. *See* Huang W, Wang T, Zong H, Zhang Y, Efficacy and Safety of Tension-Free Vaginal Tape-Secure Mini-Sling Versus Standard Midurethral Slings for Female Stress Urinary Incontinence: A Systematic Review and Meta-Analysis, International Neurourology Journal, 2015, 19(4):246-58.. *See also* Ellington DR, Erekson EA, Richter HE, Outcomes of Surgery for Stress Urinary Incontinence in the Older Woman, Clin Geriatr Med., 2015, 31(4):487-505. The FDA has issued several Public Health Notifications regarding surgical mesh placed through the vagina (transvaginal placement) to treat POP and SUI. The FDA identified serious and frequent complications with surgical mesh, including mesh erosion through the vagina, pain, infection, bleeding, discomfort during intercourse, organ perforation, urinary problems, recurrent prolapse, neuro-muscular problems, vaginal scarring/shrinkage & emotional problems. Most surgical complications require intervention including medical, additional surgical treatment and hospitalization.

Prior art pessaries, which have been most commonly used for management of female POP but also have been used for SUI, have presented a viable non-surgical option for treating SUI and POP. These prior art pessaries have had few complications and side effects. However, these devices are traditionally placed in the vagina for an extended period of time. They may also be uncomfortable. Furthermore, these prior art devices have been difficult for the patient to insert and remove, and use, insertion and removal of these devices have often required regular office visits with a physician for years. *See,* Jones KA, Harmanli O, Pessary Use in Pelvic Organ Prolapse and Urinary Incontinence, Rev Obstet Gynecol, 2010, 3(1):3-9. Difficulty with self-removal and insertion of the pessary, having the pessary fall out during defecation, and lack of comfort and convenience may be limiting widespread use of the prior art devices.

WO 2007/082341 A1 for example relates to a vaginal cup including a cup-shaped container having a principal longitudinal axis, an outer surface and an upwardly facing mouth with an associated thickened portion, said cup-shaped container including a generally frustoconical portion defining a continuous inner surface; and gripping means extending from said quasi-spherical portion, for the purpose of manually removing said cup from operative position; said cup-shaped container tending to resiliently resist bending of said container about an axis substantially perpendicular to said principal axis, while the thickened portion allows limited bending of said container about an axis substantially parallel to said principal axis.

WO 2015/041353 A1 relates to a ring pessary for the treatment of uterine prolapse or hysteroptosis, the ring pessary being used to control uterine prolapse or hysteroptosis by supporting the lower uterine segment. The ring pessary is characterized in that: the ring pessary includes a resin ring; the resin ring has an outer diameter (OD) of 5-9 cm, and the load necessary to compress the outer diameter to 2 cm is 2-10 N; and the external dimension of the resin ring seconds after the load is removed is at least 70% of said outer diameter of the resin ring.

US 2003/149334 A1 relates to a vaginal insert has a flexible body either in the form of a belt or a split cylinder. In either case, the insert may be coiled into a coiled state. The body has a reduced diameter when coiled and exhibits a resilient bias toward uncoiling and expanding in diameter from the coiled state. When the insert is inserted into the vagina of a patient, it expands and presses against the vaginal wall.

US 3 845 766 A relates to a menstrual device for internal use consisting of a liquid-proof, soft and elastomeric material. The device is generally cup-shaped and has on its outside annular ribs extending peripherally around the wall of the cup, the ribs having for its purpose to sealingly abut the wall of the vaginal canal when the device is in use in order to maintain the device in position in the vaginal canal. The invention is characterized in that at least one annular rib extending around the periphery of the wall of the cup is wave-shaped.

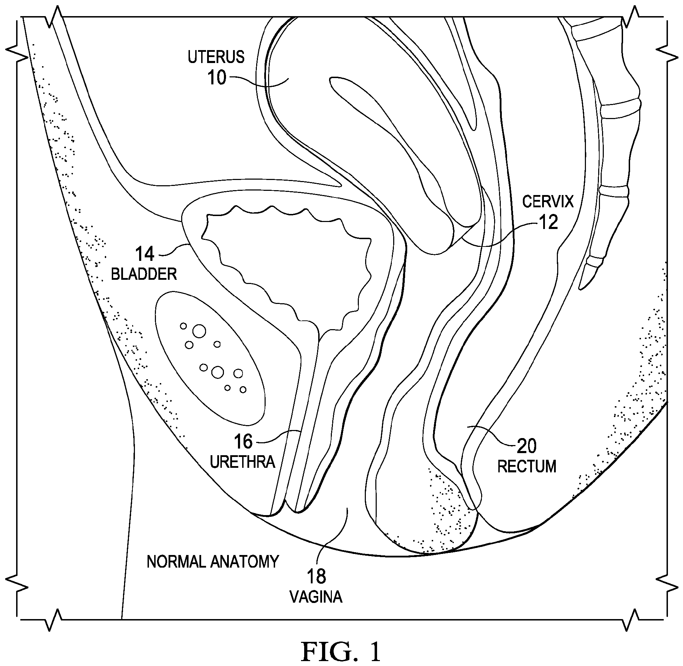

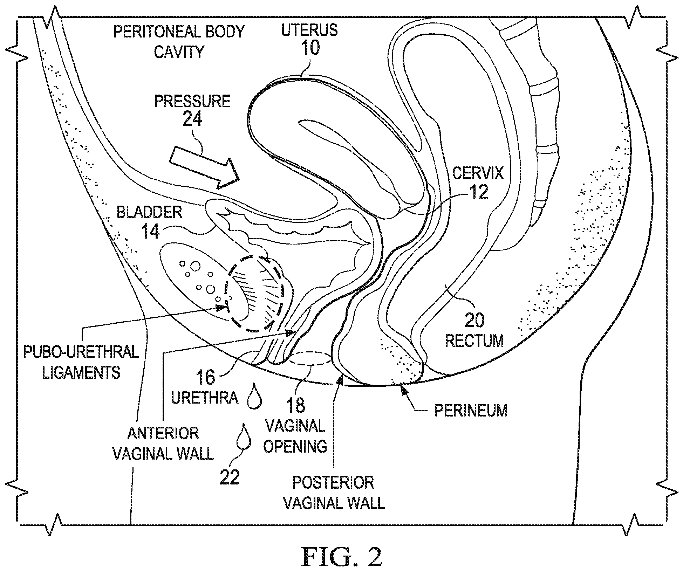

Stress Urinary Incontinence (SUI) in women, is the involuntary leakage of urine due to a weakened pelvic support system and/or pressure on the bladder from aging, genetics, or childbirth. The Urology Care Foundation estimates that one of every three women will experience SUI at some point in their lifetime. There are a few types of urinary incontinence including stress incontinence, urge incontinence and mixed incontinence. All are mainly due to connective tissue laxity or damage in the vagina or supportive ligaments. See An Integral Theory and Its Method for the Diagnosis and Management of Female Urinary Incontinence, Petros PE, Ulmsten UI, Scand J Urol Nephrol Suppl, 1993,153, 1-93. FIGURE 1 is a cross-section of the pelvic region of a female with normal anatomy illustrating the uterus 10, cervix 12, bladder 14, urethra 16, vagina 18, and rectum 20. FIGURE 2 illustrates urinary incontinence (i.e., leakage of urine) 22 caused by stress or pressure 24 on the bladder 14. Involuntary leakage of urine often occurs during activities such as coughing, laughing, sneezing, lifting or exercise.

Connective tissue damage to three zones of the Integral System, which encompasses all three pelvic organs, including the bladder, vagina and ano-rectum, is the ultimate cause of Pelvic Organ Prolapse (POP) and dysfunction in these organs. FIGURE 3 is a cross-section of the pelvic region of a female with a prolapsed bladder 26. FIGURE 4 is a cross-section of the pelvic region of a female with a prolapsed back-passage 28. FIGURE 5 is a cross-section of the pelvic region of a female with a prolapsed uterus 30. POP is commonly due to child bearing but may also be caused simply by genetics and the aging process.

Therefore, there is a need for a pessary or other vaginal insert device that manages, improves, or eliminates female incontinence, POP or both incontinence and POP. There is a further need for such a pessary or other vaginal insert device that does not require a prescription, and is non-absorbent, over the counter, convenient, comfortable, and easy for a patient to insert and remove, with no or minimal physician intervention. Preferably such a vaginal insert device would also be reusable, but it also can be disposable.

### SUMMARY

In accordance with the teachings of the present disclosure, the disadvantages and problems associated with prior art pessaries may be substantially reduced or eliminated.

In accordance with embodiments of the present disclosure, a vaginal insert device for use in improving symptoms associated with pelvic organ prolapse, urinary incontinence, or both pelvic organ prolapse and urinary incontinence can include an upper portion, which is made of an elastic and non-absorbent material, having a cone-shaped body, having a circular transverse cross-section throughout its length, having a wall with an interior side and an exterior side, an upper open end, a lower end, and a hollow interior, wherein the circumference of the upper portion decreases from the upper open end to the lower end, wherein the upper open end of the upper portion is the innermost portion of the vaginal insert device during insertion, and wherein the wall of the upper portion can be squeezed to make the upper portion more compact for easier insertion of the vaginal insert device, and wherein the wall expands back to its original shape after insertion. Such vaginal insert device can further include: an exterior rim surrounding and protruding from the exterior side of the wall of the upper portion and being adjacent to the upper open end; and a plurality of ridges surrounding and protruding from the exterior side of the wall of the upper portion and being spaced apart from the upper open end to the lower end.

In accordance with these and other embodiments of the present disclosure, a vaginal insert device can include a removal portion extending from the lower end of the upper portion, wherein the removal portion can be accessed from the exterior of a vagina when the vaginal insert device is inserted in the vagina, and wherein the removal portion assists in removal of the vaginal insert device from the vagina. The removal portion can comprise a string or a stem. In the embodiment where the removal portion is a stem, the stem can extend from the lower end of the upper portion, wherein the upper portion and the stem comprise an integral one-piece device made from the elastic and non-absorbent material, wherein the stem has a cone-shaped body, having a circular transverse cross-section throughout its length, having a wall, an upper end, a lower open end, and a hollow interior, and wherein the circumference of the stem increases from the upper end to the lower open end. The removal portion can also have a plurality of ridges like the upper portion.

In accordance with these and other embodiments of the present disclosure, a vaginal insert device can include one or more ventilation openings. A ventilation hole can be located at the point where the lower end of the upper portion and a stem intersect. One or more ventilation openings can also be located in the wall on the upper portion.

In accordance with these and other embodiments of the present disclosure, a vaginal insert device can include an exterior rim which is circular and has a first section and a second section, wherein the first section protrudes from the exterior side of the wall of the upper portion a greater distance than the second section.

In accordance with these and other embodiments of the present disclosure, a vaginal insert device can include an applicator used during insertion of the device, wherein the applicator can contain at least the upper portion when it is in a more compact shape, and wherein the applicator assists in the insertion of the device.

Medical and other advantages of the present disclosure may be readily apparent to one skilled in the art from the figures, description and claims included herein. The objects and advantages of the embodiments will be realized and achieved at least by the elements, features, and combinations particularly pointed out in the claims.

It is to be understood that both the foregoing general description and the following detailed description are examples and explanatory and are not restrictive on the claims set forth in this disclosure.

### BRIEF DESCRIPTION OF THE DRAWINGS

A more complete understanding of the present embodiments and advantages thereof may be acquired by referring to the following description taken in conjunction with the accompanying drawings, in which like reference numbers indicate like features, and wherein:

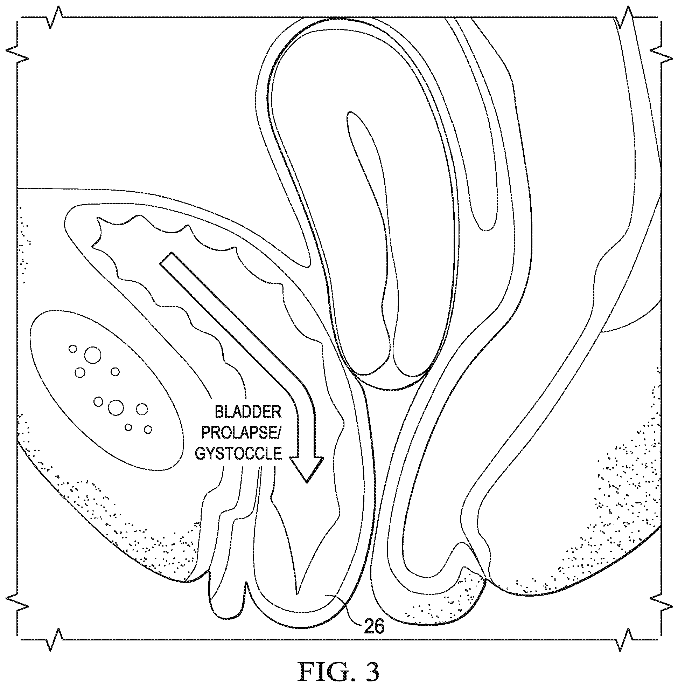

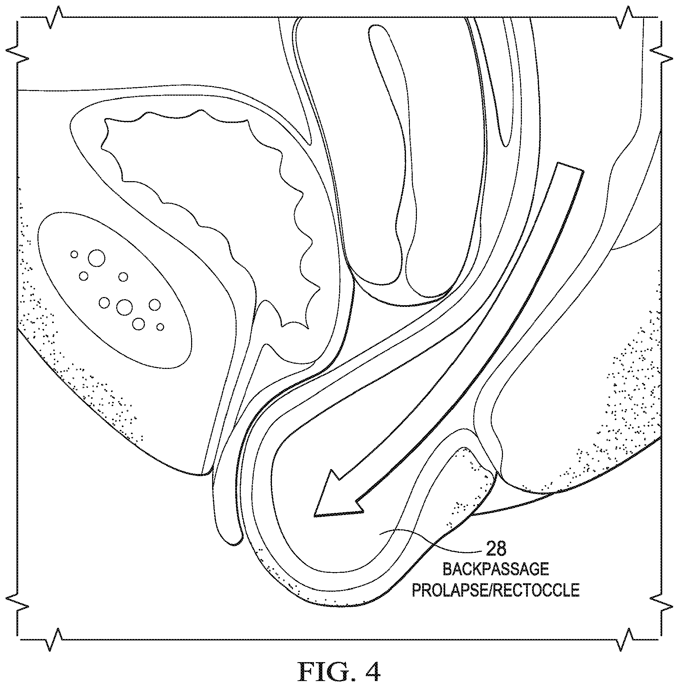

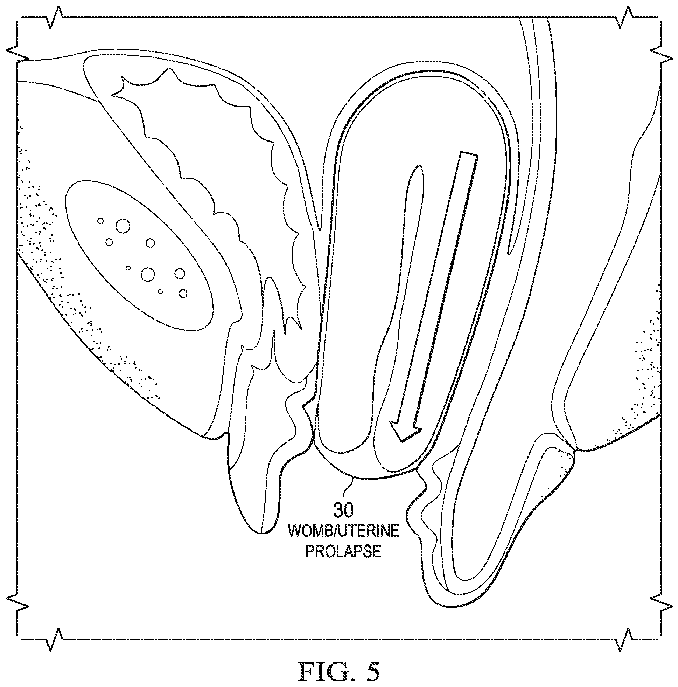

FIGURES 1-5 each illustrate a cross-section of the pelvic region of a female for purposes of discussing the background of the present disclosure;

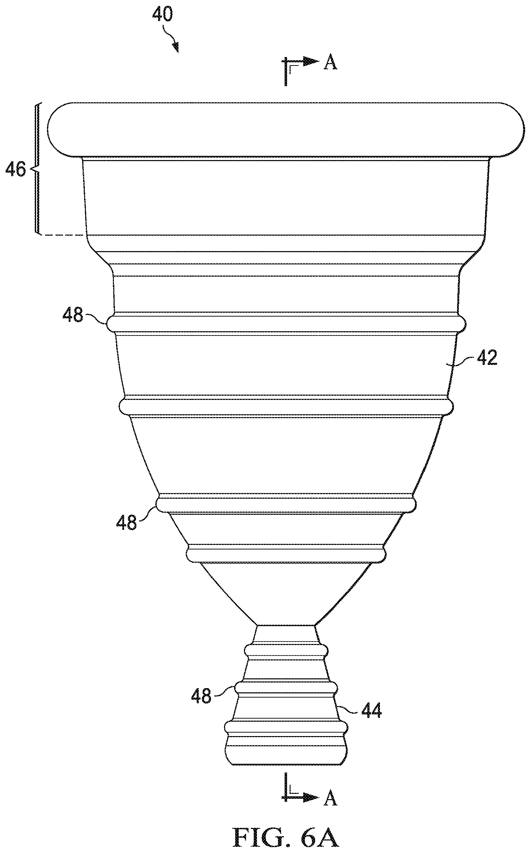

FIGURE 6A illustrates a side view of an example vaginal insert device, in accordance with embodiments of the present disclosure;

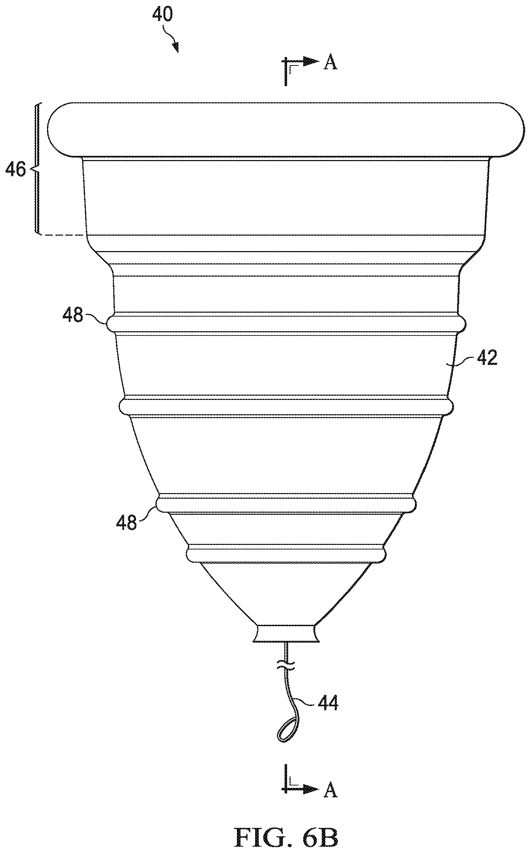

FIGURE 6B illustrates a side view of another example vaginal insert device, in accordance with embodiments of the present disclosure;

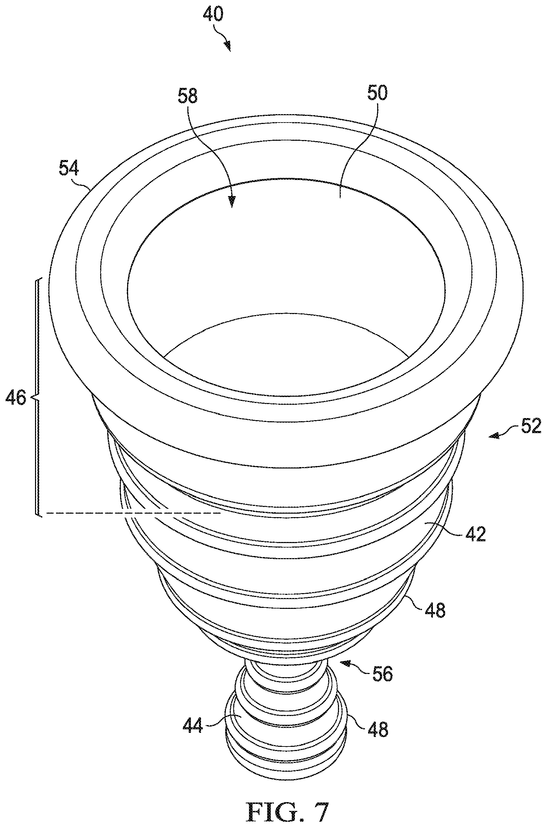

FIGURE 7 illustrates a perspective view of the vaginal insert device of FIGURE 6A;

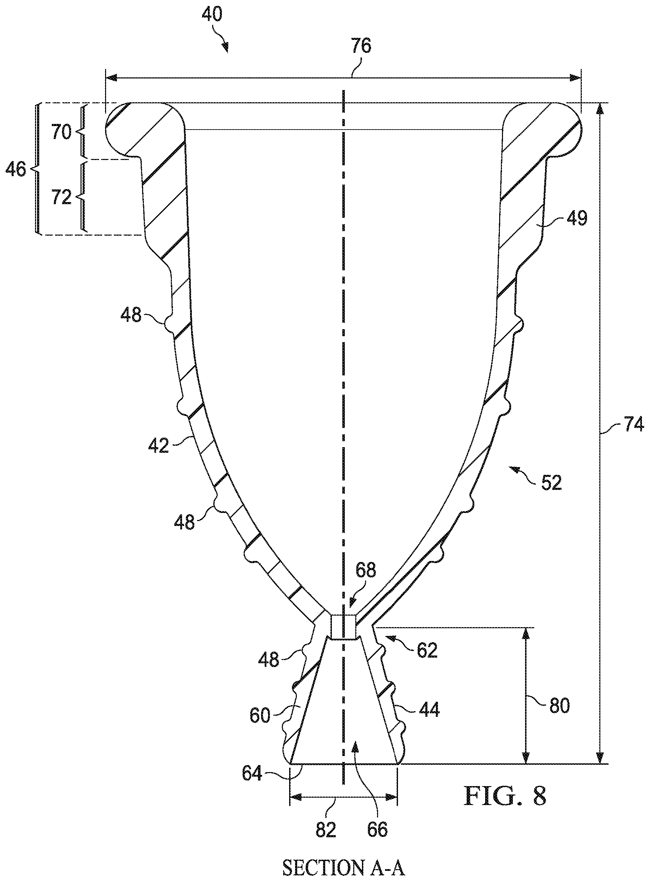

FIGURE 8 is a cross-sectional view of vaginal insert device of FIGURE 6A across section line A-A;

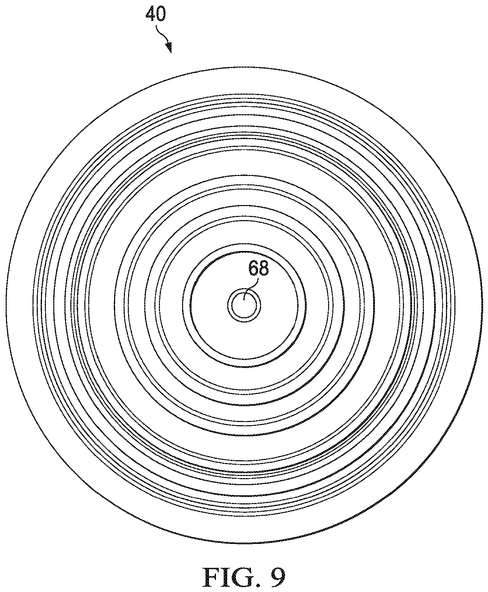

FIGURE 9 is a bottom view of the vaginal insert device of FIGURE 6A;

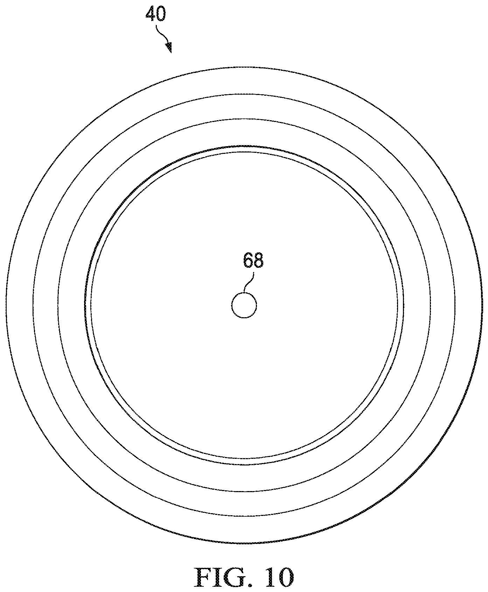

FIGURE 10 is a top view of the vaginal insert device of FIGURE 6A;

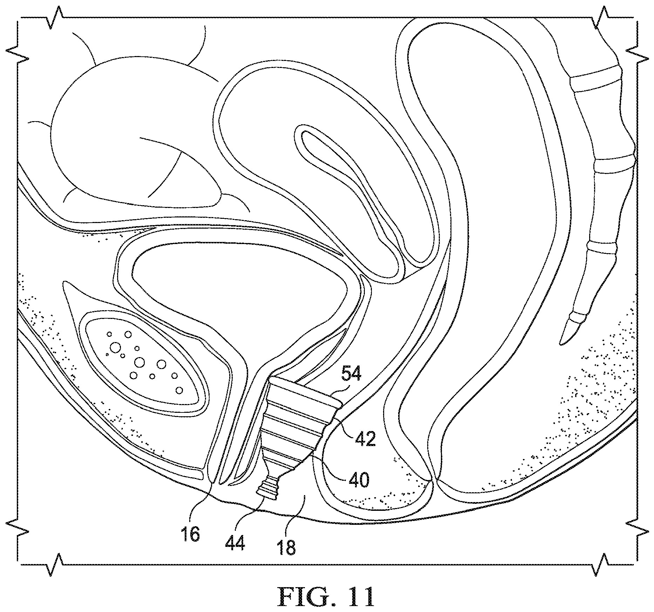

FIGURES 11-13 each illustrate a cross-section of the pelvic region of a female as well as an embodiment of the vaginal insert device inserted in the vagina;

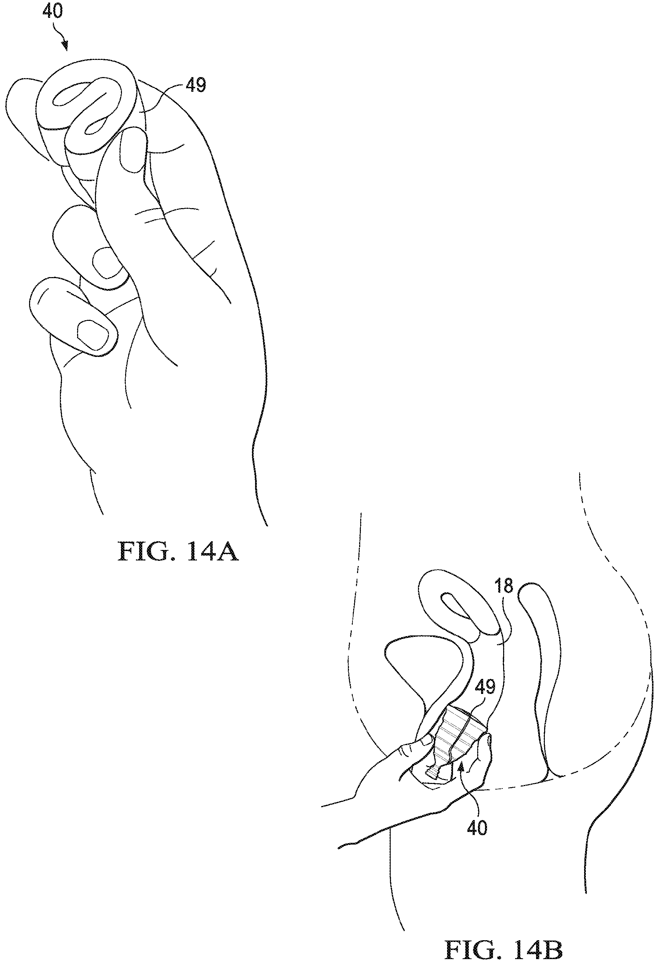

FIGURES 14A and 14B illustrate a method for inserting an embodiment of the vaginal insert device into a patient's vagina;

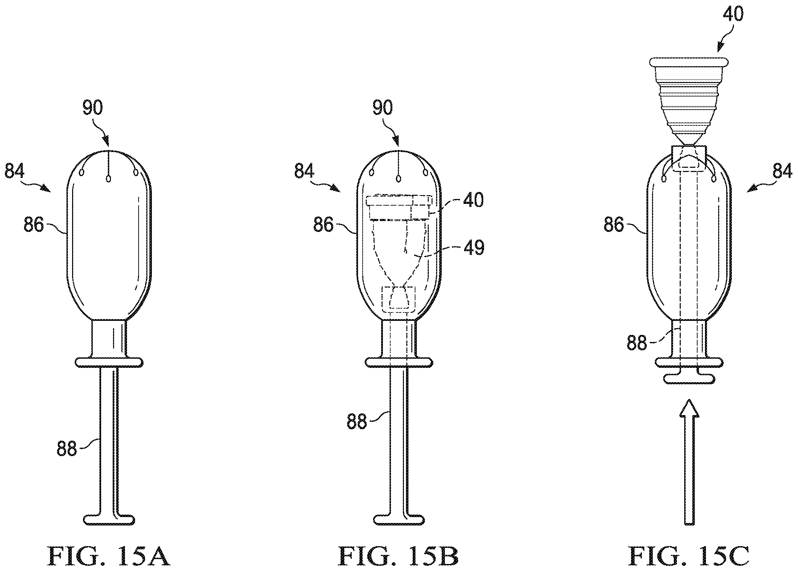

FIGURE 15A illustrates a side-view of a pessary applicator; and
FIGURES 15B-15C illustrate a cross-section of the pessary applicator of FIGURE 15A, and a method of using same to insert an embodiment of the vagina insert device into a patient's vagina.

### DETAILED DESCRIPTION

Preferred embodiments and their advantages are best understood by reference to FIGURES 6A through 15C, wherein like numbers are used to indicate like and corresponding parts or sections.

The vaginal insert device of the present invention manages, improves, or eliminates female urinary incontinence, Pelvic Organ Prolapse (POP), or both POP and urinary incontinence. It does not require a prescription, and is non-implantable, non-absorbent, over the counter, convenient, flexible, comfortable, and easy for a patient to insert and remove, with no or minimal physician intervention. Preferably such a vaginal insert device would also be reusable, but it also can be disposable. It eliminates concern of Toxic Shock Syndrome (TSS) by not consisting of an absorbency element which could produce odor and breed bacteria. The device of the present invention is fabricated, such as by a molding process, with an elastic and non-absorbent material, preferably a biocompatible elastomer such as medical grade silicone. An example of a silicone material suitable for use in the present invention is NuSil Technology's MED-4950 product, which is characterized as a liquid silicone rubber.

The vaginal insert device of the present invention is shaped so that it is held securely in place in the vagina when inserted, as well as shaped to impose pressure on the urethral sphincter and to support pelvic organs. One exemplary use of the vaginal insert device of the present invention is for management of stress urinary incontinence (SUI) and the involuntary leakage of urine during activities such as coughing, laughing, sneezing, lifting and exercise for patients over the age of eighteen. It is expected that the device will typically be inserted by an adult woman for up to about twelve hours or more, depending on their comfort level.

FIGURE 6A illustrates a side view of an example vaginal insert device 40, in accordance with embodiments of the present disclosure. The vaginal insert device 40 of FIGURE 6A comprises an upper portion 42 and a lower, stem-like, removal portion 44. As further illustrated and described in more detail below, the upper portion 42 includes a rim 46 as well as ridges 48 which are preferably spaced apart from the top of the upper portion to the lower end of the upper portion. Ridges 48 can be randomly or uniformly spaced. The removal portion can also have ridges 48, which like the upper portion are spaced apart from the top of the removal portion to the lower end of the removal portion.

FIGURE 6B illustrates a side view of another example vaginal insert device 40, in accordance with embodiments of the present disclosure. In this embodiment, device 40 is the same as the embodiment shown in FIGURE 6A except the removal portion 44 is a string, cord or ribbon (collectively referred to as "a string"), such as is used in removing a tampon.

FIGURE 7 illustrates a perspective view of the vaginal insert device 40 of FIGURE 6A. As shown in FIGURE 7, the upper portion 42 has a cone-shaped body, having a circular transverse cross-section throughout its length, has a wall 49 with an interior side 50 and an exterior side 52, an upper open end 54, a lower end 56, and a hollow interior 58, wherein the circumference of the upper portion decreases from the upper open end to the lower end. As illustrated, rim 46 is preferably circular and surrounds and protrudes from the exterior side 52 of the wall and is adjacent to the upper open end 54. Ridges 48 are preferably circular rings which surround the exterior side 52 of wall 49. As further illustrated, a stem or removal portion 44 extends from the lower end 56 of the upper portion 42. The upper portion 42 and the stem 44 can be fabricated as an integral one-piece device. FIGURE 7 also illustrates that the stem 44 can have a cone-shaped body.

FIGURE 8 is a cross-sectional view of vaginal insert device 40 of FIGURE 6A across section line A-A. As shown in FIGURE 8, stem 44 can have a circular transverse cross-section throughout its length, having a wall 60, an upper end 62, a lower open end 64, and a hollow interior 66, wherein the circumference of the stem increases from the upper end to the lower open end. As illustrated, ridges 48 protrude from the exterior side 52 of the wall 49.

FIGURE 8 further illustrates an embodiment of vaginal insert device 40 which includes a ventilation opening 68. The ventilation opening can include a ventilation hole, a screen or a mesh or any other component with an opening. As would be understand by one of ordinary skill of the art, vaginal insert device 40 can include a plurality of ventilation openings in locations other than, or in addition to, ventilation opening 68, such as in wall 49 of the upper portion 42. The one or more ventilation openings are intended to make the vaginal insert device more comfortable for the patient when inserted. The one or more openings may also equalize the air pressure between the inside and outside of the vagina 18, when vaginal insert device 40 is inserted. However, having a ventilation opening is not required for the vaginal insert device to be comfortable, useful and effective.

FIGURE 8 illustrates further detail regarding an embodiment where rim 46 has a first section 70 and a second section 72, both of which protrude from the exterior side 52 of the wall 49, with the first section protruding a greater distance than the second section. A person of ordinary skill in the art would understand that multiple alternative embodiments of rim 46 are possible, including an embodiment in which rim 46 comprises just the first section 70, or another embodiment in which rim 46 comprises only one section but has a greater height than first section 70 and protrudes from the exterior side 52 of the wall the same distance from the top of the rim to the bottom of the rim. In another embodiment, rim 46 surrounds the exterior side 52 of the wall 49, adjacent to the upper open end 54, and comprises sections which protrude and sections which do not protrude from the exterior side of the wall.

With reference to FIGURES 7 and 8, the term "ridges" is intended to be broadly defined. Accordingly, ridges 48 can include the embodiment illustrated in FIGURES 7 and 8, in which ridges 48 are protrusions which are preferably circular rings which surround the exterior side of wall 49. However, "ridges" can also include any protrusions that extend from the exterior side 52 of wall 49, such as a plurality of studs or knobs.

A person of ordinary skill in the art would understand that the vaginal insert device 40 can come in different sizes, including different sizes to accommodate adult women with differing anatomy. Furthermore, a person of ordinary skill in the art would understand that the dimensions of the various sections and portions of the device 40 can be modified from the multiple embodiments illustrated and disclosed herein. For example, referring to Figure 8, the total height 74 of device 40, diameter 76 at the upper open end 54 of the upper portion 42, and thickness of the wall 49 of the upper portion 42 of the device 40 can be modified, while retaining or improving on the intended usefulness, effectiveness and other benefits of the device. The following non-exclusive list of dimensions, in reference to FIGURE 8, are non-limiting examples of embodiments of device 40 which are believed to be suitable for most women, and which provide the intended usefulness, effectiveness and other benefits of the device. For example, a suitable total height of device 40 is estimated to be in the range of about 58.0 to 67.0 millimeters (mm), and a suitable outer diameter 76 is estimated to be in the range of about 38.0 to 44.0 mm. A suitable height of the stem 44 is estimated to be in about the 13.0 to 15.0 mm range. A suitable thickness of the wall 49, in sections without ridges 48, of the upper portion 42, below rim 46, is estimated to be about 2.0 mm, whereas a suitable thickness of the wall 49, in sections with ridges is estimated to be about 2.5 mm. Wall 60 of stem 44 is estimated to have a suitable thickness of about 1.25 mm in sections without ridges 48. A suitable height of rim 46 is estimated to be about 15.0 mm, with a suitable height of first section 70 estimated to be about 5.0 mm and a suitable height of second section 72 estimated to be about 10.0 mm. A suitable thickness of the first section 70 is estimated to be about 6.0 mm and the second section 72 is estimated to be about 4.0 mm. A suitable outer diameter 82 of lower open end 64 is estimated to be about 10.5 mm. A suitable diameter of ventilation opening 68 is estimated to be about 2.0 mm.

FIGURE 9 is a bottom view of the vaginal insert device 40 of FIGURE 6A for an embodiment having ventilation opening 68. FIGURE 10 is a top view of the vaginal insert device 40 of FIGURE 6A for an embodiment having ventilation opening 68.

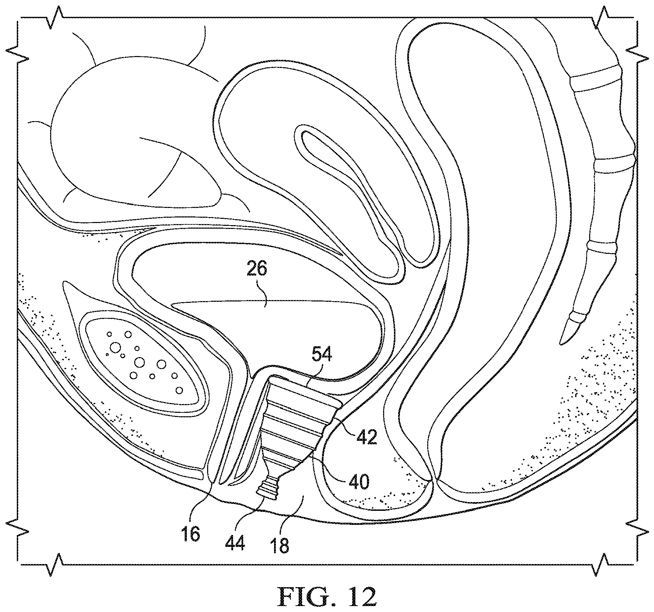

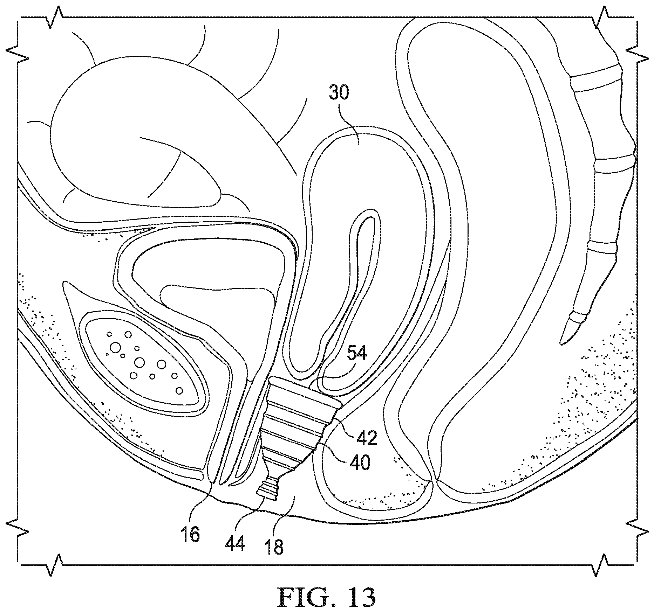

FIGURE 11 is a cross-section of the pelvic region of a female illustrating an embodiment of the vaginal insert device 40 inserted in the vagina 18 and applying pressure on the urethral sphincter 16 to manage, improve, or eliminate female urinary incontinence. FIGURES 12 and 13 are each a cross-section of the pelvic region of a female illustrating an embodiment of the vaginal insert device 40 inserted in the vagina 18 to manage, improve, or eliminate POP, in addition to applying pressure on the urethral sphincter 16 to manage, improve, or eliminate female urinary incontinence. In particular, in FIGURE 12, vaginal insert device 40 is inserted in the vagina 18 to manage, improve, or eliminate a prolapsed bladder 26. In particular, in FIGURE 13, vaginal insert device 40 is inserted in the vagina 18 to manage, improve, or eliminate a prolapsed uterus 30. As illustrated in FIGURES 11-13, the upper open end 54 of the upper portion 42 is the innermost portion of the vaginal insert device 40 during insertion. As further illustrated in FIGURES 11-13, the removal portion or stem 44 of an embodiment of vaginal insert device 40 can be accessed from the exterior of the vagina 18 when the vaginal insert device is inserted and assists in removal of the vaginal insert device. Ridges 48 on stem 44 provide better grip for removal of the device 40 by a patient.

FIGURES 14A and 14B illustrate a method for comfortably inserting vaginal insert device 40 into vagina 18. As illustrated in FIGURE 14A, a patient can manually squeeze the wall 49 of the upper portion to make the upper portion more compact for easier insertion of the vaginal insert device 40. As illustrated in FIGURE 14B, once vaginal insert device 40 is manually inserted into vagina 18, wall 49 expands back to its original shape.

FIGURE 15A illustrates a side-view of a pessary applicator 84 which can be used to assist in inserting a vaginal insert device 40 into a patient's vagina 18. Pessary applicator 84 comprises an insertion member 86, a top portion 90 of the insertion member, and a plunger 88. Pessary applicator 84 is generally similar to a tampon applicator. However, the insertion member 86 will generally have a greater circumference than a tampon applicator to accommodate a vaginal insert device 40, which when compacted has a shape which is generally larger than the circumference of a tampon. FIGURES 15B-15C illustrate a cross-section of the pessary applicator 84 of FIGURE 15A, and a method of using the applicator to comfortably insert a vaginal insert device 40 into a patient's vagina 18. As is illustrated, vagina insert device 40 is housed inside insertion member 86 in a compacted shape. Similar to the process of insertion of a tampon into a vagina using an applicator, insertion member 86 of applicator 84 is inserted into the patient's vagina 18, and the plunger 88 is pushed towards the insertion member, ejecting the vaginal insert device 40 through the top portion 90. The applicator 84 is then removed from the vagina 18, and the vaginal insert device 40 remains in place in the vagina, expanded back to its normal shape. The vaginal insert device 40 which has been inserted is positioned such as is illustrated in FIGURE 11.

This disclosure encompasses all changes, substitutions, variations, alterations, and modifications to the example embodiments herein that a person having ordinary skill in the art would comprehend, as long as they fall within the scope of the appended claims.

## Claims

1. A vaginal insert device (40) for use in improving symptoms associated with pelvic organ prolapse, urinary incontinence, or both pelvic organ prolapse and urinary incontinence, comprising:
an upper portion (42), which is made of an elastic and non-absorbent material, having a cone-shaped body, having a circular transverse cross-section throughout a length of the upper portion (42), having a wall (49) with an interior side (50) and an exterior side (52), an upper open end (54), a lower end (56), and a hollow interior (58), wherein a circumference of the upper portion decreases from the upper open end to the lower end, wherein the upper open end of the upper portion is the innermost portion of the vaginal insert device during insertion, and wherein the wall (49) of the upper portion is configured to be squeezed to make the upper portion into a more compact shape for easier insertion of the vaginal insert device, and wherein said wall (49) expands back to its original shape after the insertion;
an exterior rim (46) surrounding and protruding from the exterior side (52) of the wall (49) of the upper portion (42), the exterior rim (46) being adjacent to said upper open end (54);
a plurality of ridges (48) surrounding and protruding from the exterior side (52) of the wall (49) of the upper portion (42), the plurality of ridges (48) being spaced apart from the upper open end (54) to the lower end (56);
wherein said upper portion (42), with said exterior rim (46) and said plurality of ridges (48), holds the vaginal insert device (40) securely in place to impose pressure on the urethral sphincter, or to support pelvic organs, or to both impose pressure on the urethral sphincter and support pelvic organs, when the device (40) is inserted.

2. The vaginal insert device of Claim 1, further comprising a stem (44) or a string (44) attached to said upper portion (42), the stem (44) or string (44) extending from said lower end (56) of said upper portion (42), wherein the stem (44) or string (44) is configured to be accessed from the exterior of a vagina when the vaginal insert device (40) is inserted in said vagina wherein the stem (44) or string (44) is configured to assist in removal of the vaginal insert device (40).

3. The vaginal insert device of Claim 1, further comprising a removal portion (44) extending from said lower end (56) of said upper portion (42), wherein the removal portion (44) is configured to be accessed from the exterior of a vagina when the vaginal insert device (40) is inserted in said vagina, and wherein said removal portion (44) assists in removal of the vaginal insert device (40).

4. The vaginal insert device of Claim 2, wherein the upper portion (42) and the stem (44) comprise an integral one-piece device made from said elastic and non-absorbent material.

5. The vaginal insert device of any of the preceding claims, wherein said material is a biocompatible elastomer.

6. The vaginal insert device of Claim 5, wherein said material is a silicone elastomer.

7. The vaginal insert device of Claim 2, wherein the upper portion (42) is a biocompatible silicone elastomer material,
wherein the upper portion (42) and the stem (44) comprise an integral one-piece device made from said silicone, and
the vaginal insert device (40) further comprising a ventilation opening (68) located at a point where the lower end (56) of the upper portion (42) and said stem (44) intersect.

8. The vaginal insert device of any of the preceding claims,
(i) further comprising a ventilation opening (68) located in the wall (49) of the upper portion (42), in the wall (49) or in the lower end (56) of the upper portion (42),
or
(ii) wherein the ventilation opening (68) has a diameter of about 2.0 mm.

9. The vaginal insert device of any of the preceding claims, further comprising a stem (44) and wherein the circumference of the upper open end (54) of the upper portion (42) is greater than a circumference of the lower end of the stem (44).

10. The vaginal insert device of any of the preceding claims, further comprising an applicator (84) used during the insertion of the device (40), wherein the applicator (84) is configured to house at least the upper portion (42) when the upper portion (42) is in said more compact shape, and wherein the applicator (84) assists in the insertion of the device (40).

11. The vaginal insert device of any of the preceding claims, wherein said exterior rim (46) is circular and has a first section (70) and a second section (72), and wherein said first section (70) protrudes from the exterior side (52) of the wall (49) of the upper portion (42) a greater distance than the second section (72).

12. The vaginal insert device of any of the preceding claims, wherein the vaginal insert device (40) comprises at least one ventilation opening (68) configured to equalize air pressure between an inside and an outside of the vagina, when the vaginal insert device (40) is inserted.

13. The vaginal insert device of any of the preceding claims, wherein the plurality of ridges (48) are distributed along an entire length of the upper portion (42) from the lower end (56) to the exterior rim (46).

14. The vaginal insert device of Claim 13, wherein the plurality of ridges (48) are uniformly distributed from the lower end (56) of the upper portion (42) to the exterior rim (46) of the upper portion (42).

15. The vaginal insert device of any of the preceding claims, wherein, in the expanded position, the exterior rim (46) is configured to impose pressure on the bladder neck at the urethral sphincter.

## Patentansprüche

1. Vaginaleinsatzvorrichtung (40) zur Verwendung für ein Verbessern von Symptomen, die einem Beckenorganprolaps, einer Harninkontinenz oder sowohl dem Beckenorganprolaps als auch der Harninkontinenz zugeordnet sind, umfassend:
einen oberen Abschnitt (42), der aus einem elastischen und nicht absorbierenden Material hergestellt ist, der einem konischen Körper aufweist, der über eine Länge des oberen Abschnitts (42) einen kreisförmigen transversalen Querschnitt aufweist, der eine Wand (49) mit einer Innenseite (50) und einer Außenseite (52), ein oberes offenes Ende (54), ein unteres Ende (56) und einen hohlen Innenraum (58) aufweist, wobei ein Umfang des oberen Abschnitts von dem oberen offenen Ende zu dem unteren Ende abnimmt, wobei das obere offene Ende des oberen Abschnitts der innerste Abschnitt der Vaginaleinsatzvorrichtung während eines Einsetzens ist, und wobei die Wand (49) des oberen Abschnitts konfiguriert ist, um zusammengedrückt zu werden, um den oberen Abschnitt in eine kompaktere Form zum leichteren Einsetzen der Vaginaleinsatzvorrichtung zu bekommen, und wobei die Wand (49) nach dem Einsetzen wieder in ihre ursprüngliche Form expandiert;
einen Außenrand (46), der von der Außenseite (52) der Wand (49) des oberen Abschnitts (42) vorsteht und diese umgibt, wobei der Außenrand (46) angrenzend an das obere offene Ende (54) ist;
eine Vielzahl von Rillen (48), die von der Außenseite (52) der Wand (49) des oberen Abschnitts (42) vorstehen und diese umgeben, wobei die Vielzahl von Rillen (48) von dem oberen offenen Ende (54) zu dem unteren Ende (56) beabstandet sind;
wobei der obere Abschnitt (42), mit dem Außenrand (46) und der Vielzahl von Rillen (48), die Vaginaleinsatzvorrichtung (40) sicher an Ort und Stelle hält, um Druck auf den Blasenschließmuskel auszuüben, oder um Beckenorgane zu unterstützen, oder um sowohl Druck auf den Blasenschließmuskel auszuüben als auch die Beckenorgane zu unterstützen, wenn die Vorrichtung (40) eingesetzt ist.

2. Vaginaleinsatzvorrichtung nach Anspruch 1, ferner umfassend einen Schaft (44) oder eine Kette (44), der/die an dem oberen Abschnitt (42) angebracht ist, wobei sich der Schaft (44) oder die Kette (44) von dem unteren Ende (56) des oberen Abschnitts (42) erstreckt, wobei der Schaft (44) oder die Kette (44) konfiguriert ist, um von der Außenseite einer Vagina zugänglich zu sein, wenn die Vaginaleinsatzvorrichtung (40) in die Vagina eingesetzt ist, wobei der Schaft (44) oder die Kette (44) konfiguriert ist, um ein Entfernen der Vaginaleinsatzvorrichtung (40) zu vereinfachen.

3. Vaginaleinsatzvorrichtung nach Anspruch 1, ferner umfassend einen Entfernungsabschnitt (44), der sich von dem unteren Ende (56) des oberen Abschnitts (42) erstreckt, wobei der Entfernungsabschnitt (44) konfiguriert ist, um von der Außenseite einer Vagina zugänglich zu sein, wenn die Vaginaleinsatzvorrichtung (40) in die Vagina eingesetzt ist, und wobei der Entfernungsabschnitt (44) das Entfernen der Vaginaleinsatzvorrichtung (40) vereinfacht.

4. Vaginaleinsatzvorrichtung nach Anspruch 2, wobei der obere Abschnitt (42) und der Schaft (44) eine einstückige einteilige Vorrichtung umfassen, die aus dem elastischen und nicht absorbierenden Material hergestellt ist.

5. Vaginaleinsatzvorrichtung nach einem der vorstehenden Ansprüche, wobei das Material ein biokompatibles Elastomer ist.

6. Vaginaleinsatzvorrichtung nach Anspruch 5, wobei das Material ein Silikonelastomer ist.

7. Vaginaleinsatzvorrichtung nach Anspruch 2, wobei der obere Abschnitt (42) ein biokompatibles Silikonelastomermaterial ist,
wobei der obere Abschnitt (42) und der Schaft (44) eine einstückige einteilige Vorrichtung umfassen, die aus dem Silikon hergestellt ist, und
die Vaginaleinsatzvorrichtung (40) ferner umfassend eine Belüftungsöffnung (68), die sich an einem Punkt befindet, wo sich das untere Ende (56) des oberen Abschnitts (42) und der Schaft (44) schneiden.

8. Vaginaleinsatzvorrichtung nach einem der vorstehenden Ansprüche,
(i) ferner umfassend eine Belüftungsöffnung (68), die sich in der Wand (49) des oberen Abschnitts (42), in der Wand (49) oder in dem unteren Ende (56) des oberen Abschnitts (42) befindet, oder
(ii) wobei die Belüftungsöffnung (68) einen Durchmesser von etwa 2,0 mm aufweist.

9. Vaginaleinsatzvorrichtung nach einem der vorstehenden Ansprüche, ferner umfassend einen Schaft (44), und wobei der Umfang des oberen offenen Endes (54) des oberen Abschnitts (42) größer als ein Umfang des unteren Endes des Schafts (44) ist.

10. Vaginaleinsatzvorrichtung nach einem der vorstehenden Ansprüche, ferner umfassend einen Applikator (84), der während des Einsetzens der Vorrichtung (40) verwendet wird, wobei der Applikator (84) konfiguriert ist, um mindestens den oberen Abschnitt (42) aufzunehmen, wenn der obere Abschnitt (42) in der kompakteren Form ist, und wobei der Applikator (84) das Einsetzen der Vorrichtung (40) vereinfacht.

11. Vaginaleinsatzvorrichtung nach einem der vorstehenden Ansprüche, wobei der Außenrand (46) kreisförmig ist und einen ersten Bereich (70) und einen zweiten Bereich (72) aufweist, und wobei der erste Abschnitt (70) von der Außenseite (52) der Wand (49) des oberen Abschnitts (42) einen größeren Abstand als der zweite Bereich (72) vorsteht.

12. Vaginaleinsatzvorrichtung nach einem der vorstehenden Ansprüche, wobei die Vaginaleinsatzvorrichtung (40) mindestens eine Belüftungsöffnung (68) umfasst, die konfiguriert ist, um einen Luftdruck zwischen einer inneren Seite und einer äußeren Seite der Vagina auszugleichen, wenn die Vaginaleinsatzvorrichtung (40) eingesetzt ist.

13. Vaginaleinsatzvorrichtung nach einem der vorstehenden Ansprüche, wobei die Vielzahl von Rillen (48) entlang einer gesamten Länge des oberen Abschnitts (42) von dem unteren Ende (56) zu dem Außenrand (46) verteilt sind.

14. Vaginaleinsatzvorrichtung nach Anspruch 13,
wobei die Vielzahl von Rillen (48) von dem unteren Ende (56) des oberen Abschnitts (42) zu dem Außenrand (46) des oberen Abschnitts (42) gleichmäßig verteilt sind.

15. Vaginaleinsatzvorrichtung nach einem der vorstehenden Ansprüche, wobei, in der expandierten Position, der Außenrand (46) konfiguriert ist, um Druck auf den Blasenhals an dem Blasenschließmuskel auszuüben.

## Revendications

1. Dispositif d'insertion vaginale (40) destiné à être utilisé pour améliorer les symptômes associés au prolapsus d'organe pelvien, l'incontinence urinaire, ou à la fois le prolapsus d'organe pelvien et l'incontinence urinaire, comprenant :
une partie supérieure (42), qui est faite d'un matériau élastique et non absorbant, ayant un corps en forme de cône, ayant une section transversale circulaire sur toute une longueur de la partie supérieure (42), ayant une paroi (49) avec un côté intérieur (50) et un côté extérieur (52), une extrémité ouverte supérieure (54), une extrémité inférieure (56) et un intérieur creux (58), une circonférence de la partie supérieure diminuant de l'extrémité ouverte supérieure à l'extrémité inférieure, l'extrémité ouverte supérieure de la partie supérieure étant la partie la plus interne du dispositif d'insertion vaginale pendant l'insertion, et la paroi (49) de la partie supérieure étant conçue pour être comprimée pour rendre la partie supérieure en une forme plus compacte pour une insertion plus aisée du dispositif d'insertion vaginale, et ladite paroi (49) se dilatant à sa forme originale après l'insertion ;
un rebord extérieur (46) entourant et faisant saillie du côté extérieur (52) de la paroi (49) de la partie supérieure (42), le rebord extérieur (46) étant adjacent à ladite extrémité ouverte supérieure (54) ;
une pluralité de crêtes (48) entourant et faisant saillie du côté extérieur (52) de la paroi (49) de la partie supérieure (42), la pluralité de nervures (48) étant espacée de l'extrémité ouverte supérieure (54) à l'extrémité inférieure (56) ;
ladite partie supérieure (42), avec ledit rebord extérieur (46) et ladite pluralité de nervures (48), maintenant le dispositif d'insertion vaginale (40) solidement en place pour imposer une pression sur le sphincter urétral, ou pour supporter des organes pelviens, ou pour imposer à la fois une pression sur le sphincter urétral et supporter des organes pelviens, lorsque le dispositif (40) est inséré.

2. Dispositif d'insertion vaginale selon la revendication 1, comprenant en outre une tige (44) ou une colonne (44) fixée à ladite partie supérieure (42), la tige (44) ou la colonne (44) s'étendant à partir de ladite extrémité inférieure (56) de ladite partie supérieure (42), la tige (44) ou la colonne (44) étant conçue pour être accessible depuis l'extérieur d'un vagin lorsque le dispositif d'insertion vaginale (40) est inséré dans ledit vagin, la tige (44) ou la colonne (44) étant conçue pour aider à l'enlèvement du dispositif d'insertion vaginale (40).

3. Dispositif d'insertion vaginale selon la revendication 1, comprenant en outre une partie d'enlèvement (44) s'étendant de ladite extrémité inférieure (56) de ladite partie supérieure (42), la partie d'enlèvement (44) étant conçue pour être accessible depuis l'extérieur d'un vagin lorsque le dispositif d'insertion vaginale (40) est inséré dans ledit vagin, et ladite partie d'enlèvement (44) aidant à retirer le dispositif d'insertion vaginale (40).

4. Dispositif d'insertion vaginale selon la revendication 2, dans lequel la partie supérieure (42) et la tige (44) comprennent un dispositif monobloc fabriqué à partir dudit matériau élastique et non absorbant.

5. Dispositif d'insertion vaginale selon l'une quelconque des revendications précédentes, dans lequel ledit matériau est un élastomère biocompatible.

6. Dispositif d'insertion vaginale selon la revendication 5, dans lequel ledit matériau est un élastomère de silicone.

7. Dispositif d'insertion vaginale selon la revendication 2, dans lequel la partie supérieure (42) est un matériau élastomère de silicone biocompatible,
la partie supérieure (42) et la tige (44) comprenant un dispositif monobloc fabriqué à partir de ladite silicone, et
le dispositif d'insertion vaginale (40) comprenant en outre une ouverture de ventilation (68) située à un point où l'extrémité inférieure (56) de la partie supérieure (42) et ladite tige (44) se croisent.

8. Dispositif d'insertion vaginale selon l'une quelconque des revendications précédentes,
(i) comprenant en outre une ouverture de ventilation (68) située dans la paroi (49) de la partie supérieure (42), dans la paroi (49) ou dans l'extrémité inférieure (56) de la partie supérieure (42), ou
(ii) dans lequel l'ouverture de ventilation (68) a un diamètre d'environ 2,0 mm.

9. Dispositif d'insertion vaginale selon l'une quelconque des revendications précédentes, comprenant en outre une tige (44) et dans lequel la circonférence de l'extrémité ouverte supérieure (54) de la partie supérieure (42) est supérieure à une circonférence de l'extrémité inférieure de la tige (44).

10. Dispositif d'insertion vaginale selon l'une quelconque des revendications précédentes, comprenant en outre un applicateur (84) utilisé pendant l'insertion du dispositif (40), dans lequel l'applicateur (84) est conçu pour loger au moins la partie supérieure (42) lorsque la partie supérieure (42) est dans ladite forme plus compacte, et l'applicateur (84) aidant à l'insertion du dispositif (40).

11. Dispositif d'insertion vaginale selon l'une quelconque des revendications précédentes, dans lequel ledit rebord extérieur (46) est circulaire et a une première section (70) et une seconde section (72), et dans lequel ladite première section (70) fait saillie depuis le côté extérieur (52) de la paroi (49) de la partie supérieure (42) à une distance supérieure à la seconde section (72).

12. Dispositif d'insertion vaginale selon l'une quelconque des revendications précédentes, dans lequel le dispositif d'insertion vaginale (40) comprend au moins une ouverture de ventilation (68) conçue pour égaliser la pression d'air entre un intérieur et un extérieur du vagin, lorsque le dispositif d'insertion vaginale (40) est inséré.

13. Dispositif d'insertion vaginale selon l'une quelconque des revendications précédentes, dans lequel la pluralité de nervures (48) est répartie sur toute une longueur de la partie supérieure (42) de l'extrémité inférieure (56) au rebord extérieur (46).

14. Dispositif d'insertion vaginale selon la revendication 13,
dans lequel la pluralité de nervures (48) est uniformément répartie de l'extrémité inférieure (56) de la partie supérieure (42) au rebord extérieur (46) de la partie supérieure (42).

15. Dispositif d'insertion vaginale selon l'une quelconque des revendications précédentes, dans lequel, dans la position déployée, le rebord extérieur (46) est conçu pour imposer une pression sur le col de la vessie au niveau du sphincter urétral.
